# EUROPEAN PATENT APPLICATION

(11) **EP 4 130 230 A1**
(43) Date of publication of application: **08.02.2023**
(21) Application number: 21776844.9
(22) Date of filing: 19.03.2021
(51) Int. Cl.: C12M 1/107

(54) **METHANE GENERATING DEVICE**

(30) Priority: 24.03.2020 JP 2020052932
(71) Applicant: Yokogawa Electric Corporation, Musashino-shi Tokyo 180-8750 (JP); National University Corporation Shizuoka University, Shizuoka-shi, Shizuoka 422-8529 (JP)
(72) Inventor: KAWANO, Makoto, Musashino-shi, Tokyo 180-8750 (JP); KIMURA, Hiroyuki, Shizuoka-shi, Shizuoka 422-8529 (JP)
(74) Representative: Hoffmann Eitle
(86) International application number: PCT/JP2021/011455
(87) International publication number: WO 2021/193453

(57) **Abstract**

A methane generating device includes: a culture tank (1) in which methane generating bacteria are cultured with a culture liquid (20); a gas feed device (2) that feeds carbon dioxide and hydrogen gas required by the methane generating bacteria to the culture tank (1); and a generated gas extraction device (3) that extracts methane generated by the methane generating bacteria.

## Description

### [Technical Field]

Some aspects of the present invention relate to a methane generating device and a methane generating method.

Priority is claimed on Japanese Patent Application No. 2020-052932, filed March 24, 2020, the content of which is incorporated herein by reference.

### [Background Art]

The following Patent Literature 1 discloses a method for synthesizing methane by reacting carbon dioxide with hydrogen for the purpose of immobilizing carbon dioxide in order to solve the problem of global warming. This method includes a first reaction step and a second reaction step. In the first reaction step, carbon monoxide (that is, CO) and water (that is, H₂O) are generated as main components from carbon dioxide (that is, CO₂) and hydrogen gas (that is, H₂). In the second reaction step, CH₄ and H₂O are generated from CO and H₂. Further, the method includes a step of removing CO₂ and H₂O remaining unreacted in the first reaction step between the first reaction step and the second reaction step.

### [Citation List]

### [Patent Literature]

[Patent Literature 1]
Japanese Patent No. 5562873

### [Summary of Invention]

### [Technical Problem]

In a chemical method such as the above-mentioned related art, it is necessary to heat a reactor to about 500 °C when obtaining methane. Further, it is known that the reaction proceeds in two steps as shown in the following Formulas (1) and (2). Formula (1) is an endothermic reaction. Therefore, it is necessary to input energy from the outside for the reaction to proceed.

Formula (1) CO₂ + H₂ → CO + H₂O ΔH = +41 kJ/mol

Formula (2) CO + 3H₂ → CH₄ + H₂O ΔH = -206 kJ/mol

Renewable energy can also be used as an energy source. However, at present, it is realistic to use fossil fuels such as petroleum and natural gas. However, when fossil fuels are used, a large amount of carbon dioxide is discharged as the fossil fuels burn. Patent Literature 1 describes that heat generated by the reaction of Formula (2) is used for the reaction of Formula (1). This is expected to improve heat balance. However, a reaction temperature represented by Formula (1) is 460 to 550 °C, which is higher than a reaction temperature of Formula (2) of 250 to 450 °C, and it is unclear whether a sufficient amount of heat can be secured.

Some aspects of the present invention have been made in view of the above problems, and an object of the invention is to provide a methane generating device and a methane generating method capable of generating methane that is a carbon resource that is useful as an energy source and can be used as a raw material for chemical products by immobilizing carbon dioxide using hydrogen gas in a simple manner and while consuming little energy.

### [Solution to Problem]

[1] To solve the above described problem, a methane generating device according to one aspect of the present invention includes: a culture tank in which methane generating bacteria are cultured with a culture liquid; a gas feed device that feeds carbon dioxide and hydrogen gas required by the methane generating bacteria to the culture tank; and a generated gas extraction device that extracts methane generated by the methane generating bacteria.
[2] In addition, in one aspect of the present invention, the methane generating device may further include: a temperature control device that controls a temperature of the culture tank.
[3] In addition, in one aspect of the present invention, the methane generating device may further include: a gas analysis device that detects a generation state of the methane in the culture tank.
[4] In addition, in one aspect of the present invention, as the gas analysis device, a light absorption analysis device may be provided.
[5] In addition, in one aspect of the present invention, the methane generating device may further include: a culture liquid state detecting device that detects a state of the culture liquid.
[6] In addition, in one aspect of the present invention, as the culture liquid state detecting device, a pH detector may be provided.
[7] In addition, in one aspect of the present invention, as the culture liquid state detecting device, an oxidation-reduction potential detector may be provided.
[8] In addition, in one aspect of the present invention, the methane generating device may further include: a liquid analysis device that detects an existing amount of a microbial community in the culture liquid.
[9] In addition, in one aspect of the present invention, as the liquid analysis device, a light absorbance measuring device may be provided.
[10] In addition, in one aspect of the present invention, the methane generating device may further include: a pressure sensor that detects a pressure of the culture tank.
[11] In addition, in one aspect of the present invention, the methane generating device may further include: a precipitate removing device that removes a precipitate generated in the culture liquid from the culture tank.
[12] In addition, in one aspect of the present invention, as the precipitate removing device, a mechanism that removes the precipitate from a drain pipe of the culture tank may be provided.
[13] In addition, in one aspect of the present invention, the methane generating device may further include: a culture liquid input device that inputs the culture liquid into the culture tank while an anaerobic state is maintained.
[14] In addition, in one aspect of the present invention, the methane generating device may further include: a reducing agent input device that inputs a reducing agent into the culture tank in order to maintain an anaerobic state of the culture liquid.
[15] In addition, in one aspect of the present invention, the methane generating device may further include: a data processor that processes measurement data related to a culture environment of the culture tank; and a device controller that controls the culture environment of the culture tank on the basis of a processing result from the data processor.
[16] In addition, in one aspect of the present invention, the methane generating device may further include: a mechanism that stirs and mixes the culture liquid and a mixed gas containing the carbon dioxide and the hydrogen gas.
[17] In addition, in one aspect of the present invention, the gas feed device may feed an inert gas to the culture tank before culture of the methane generating bacteria begins in the culture tank.
[18] In addition, in one aspect of the present invention, the measurement data related to the culture environment of the culture tank may be an oxidation-reduction potential of the culture liquid, and when the oxidation-reduction potential is out of a range in which the methane generating bacteria can proliferate, the device controller may add a regulating agent for preparing the oxidation-reduction potential to the culture tank such that the oxidation-reduction potential is within a range in which the methane generating bacteria can proliferate.
[19] In addition, in one aspect of the present invention, the measurement data related to the culture environment of the culture tank may be a pH of the culture liquid, and when the pH is out of a range in which the methane generating bacteria can proliferate, the device controller may add a regulating agent for preparing the pH to the culture tank such that the pH is within a range in which the methane generating bacteria can proliferate.
[20] In addition, a methane generating method according to one aspect of the present invention includes: feeding carbon dioxide and hydrogen gas required by methane generating bacteria to the culture tank in which the methane generating bacteria are cultured with a culture liquid; and extracting methane generated by the methane generating bacteria.

### [Advantageous Effects of Invention]

According to some aspects of the present invention, it is possible to obtain a methane generating device and a methane generating method capable of generating methane that is a carbon resource that is useful as an energy source and can be used as a raw material for chemical products by immobilizing carbon dioxide using hydrogen gas in a simple manner and while consuming little energy.

### [Brief Description of Drawings]

Fig. 1 is a configuration diagram of a methane generating device according to an embodiment of the present invention.
Fig. 2 is a graph showing an example of a gas analysis result according to the embodiment of the present invention.

### [Description of Embodiments]

Hereinafter, an embodiment of the present invention will be described with reference to the drawings.

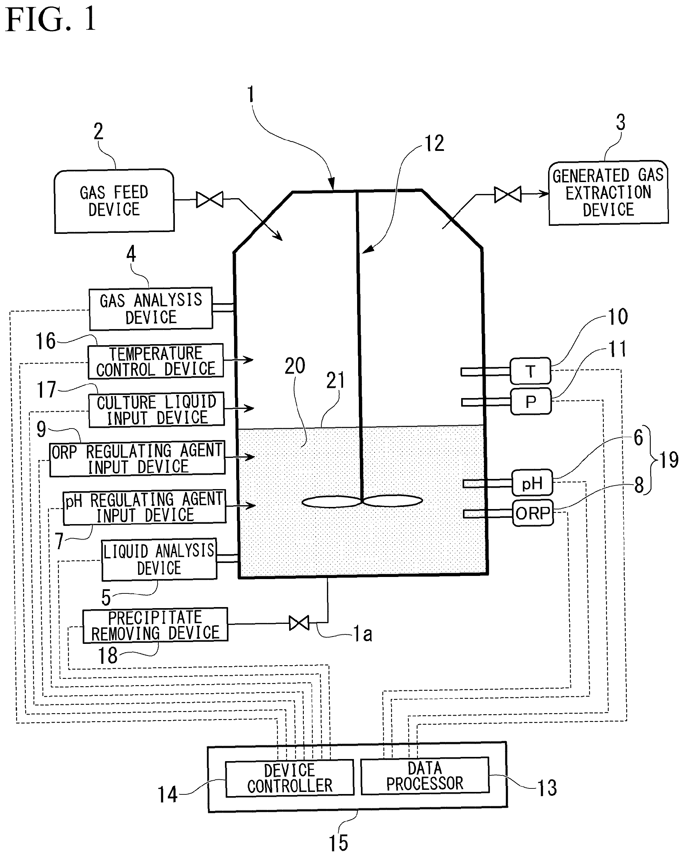

Fig. 1 is a configuration diagram of a methane generating device according to the embodiment of the present invention.

As shown in Fig. 1, the methane generating device includes a culture tank 1, a gas feed device 2, a generated gas extraction device 3, and a management device 15.

In the culture tank 1, methane generating bacteria are cultured in a culture liquid 20. Examples of the methane generating bacteria include *Methanobacterium alcaliphilum, Methanobacterium bryantii*, *Methanobacterium congolense, Methanobacterium defluvii*, *Methanobacterium espanolae*, *Methanobacterium formicicum*, *Methanobacterium ivanovii, Methanobacterium palustre, Methanobacterium thermaggregans*, *Methanobacterium uliginosum, Methanobrevibacter acididurans, Methanobrevibacter arboriphilicus, Methanobrevibacter gottschalkii, Methanobrevibacter olleyae, Methanobrevibacter ruminantium*, *Methanobrevibacter smithii, Methanobrevibacter woesei, Methanobrevibacter wolinii, Methanothermobacter marburgensis, Methanothermobacter thermoautotrophicus, Methanobacterium thermoautotrophicus, Methanothermobacter thermoflexus*, *Methanothermobacter thermophilics, Methanothermobacter wolfeii*, *Methanothermus sociabilis*, *Methanocorpusculum bavaricum*, *Methanocorpusculum parvum, Methanoculleus chikuoensis, Methanoculleus submarinus, Methanogenium frigidum, Methanogenium liminatans, Methanogenium marinum, Methanomicrobium mobile, Methanocaldococcus jannaschii, Methanococcus aeolicus, Methanococcus maripaludis*, *Methanococcus vannielii*, *Methanococcus voltaei, Methanothermococcus thermolithotrophicus*, and the like.

In the culture tank 1 of the present embodiment, strains such as *Methanobacteriales*, *Methanomicrobiales*, *Methanocellales*, and *Methanomassillicoccales* are cultured as methane generating bacteria. These hydrogen-utilizing methane generating bacteria may also be contained in anaerobic groundwater. It is possible to obtain methane by adding a mixed gas of carbon dioxide (that is, CO₂) and hydrogen gas (that is, H₂) to anaerobic groundwater containing the methane generating bacteria. That is, the culture liquid 20 (for example, the anaerobic groundwater) containing a medium for culturing the methane generating bacteria is stored in the culture tank 1. The inside of the culture tank 1 is in an anaerobic state in which the oxygen content is reduced as much as possible.

The gas feed device 2 feeds carbon dioxide and hydrogen gas required by the methane generating bacteria to the culture tank 1. The gas feed device 2 introduces a mixed gas of H₂/CO₂ (for example, 80 vol.%: 20 vol.% in volume ratio) into a gas phase portion in the culture tank 1 and pressurizes it to a predetermined pressure to begin culture of the methane generating bacteria.

The generated gas extraction device extracts the methane generated by the methane generating bacteria. The generated gas extraction device 3 takes out the generated gas from the gas phase portion in the culture tank 1 and stores it in an external tank (not shown). After the extraction of the methane is completed, the mixed gas of H₂/CO₂ is fed from the gas feed device 2 to the culture tank 1 again to perform the culture of the methane generating bacteria and the generation of the methane.

Before the culture of the methane generating bacteria begins (that is, before the mixed gas of H₂/CO₂ is fed to the culture tank 1), it is preferable to feed an inert gas into the culture tank 1 from the gas feed device 2 or the like. An anaerobic state can be created inside the culture tank 1 by feeding the inert gas (that is, gas purging). Examples of the inert gas include nitrogen gas, argon gas, helium gas, and the like.

The culture tank 1 is provided with a stirring mechanism 12 for stirring and mixing the area of a gas-liquid interface 21 between the culture liquid 20 and the mixed gas of H₂/CO₂ (that is, a reaction gas). The stirring mechanism 12 is provided with a stirring blade and makes the gas-liquid interface 21 of the culture liquid 20 ruffle. As a result, the stirring mechanism 12 promotes the dissolution of the reaction gas in the culture liquid 20. If the area of the gas-liquid interface 21 can be increased, for example, a mechanism for vibrating the culture tank 1 itself may be used instead of the stirring mechanism 12.

The management device 15 manages the above-mentioned culture tank 1, the gas feed device 2, the generated gas extraction device 3, and peripheral devices of the culture tank 1 which will be described later. The management device 15 enables long-term operation of the methane generating device (that is, long-term generation of methane). The management device 15 includes a data processor 13 that processes measurement data related to a culture environment of the culture tank 1 and a device controller 14 that controls the culture environment of the culture tank 1 on the basis of the processing result from the data processor 13.

In the present embodiment, as the peripheral devices of the culture tank 1, a gas analysis device 4, a liquid analysis device 5, a pH detector 6, a pH regulating agent input device 7, an oxidation-reduction potential (ORP) detector 8 (that is, an oxidation-reduction potential detector), an ORP regulating agent input device 9 (that is, a reducing agent input device), a temperature sensor 10, a pressure sensor 11, a temperature control device 16, a culture liquid input device 17, a precipitate removing device 18, and the like are provided.

The gas analysis device 4 is a methane detecting device that detects a generation state of the methane in the culture tank 1. Examples of a gas analysis method by the gas analysis device 4 include gas chromatography, infrared spectroscopy, Raman spectroscopy, mass analysis, and the like. The gas analysis device 4 of the present embodiment includes a light absorption analysis device that can obtain the analysis result relatively quickly in these gas analysis methods.

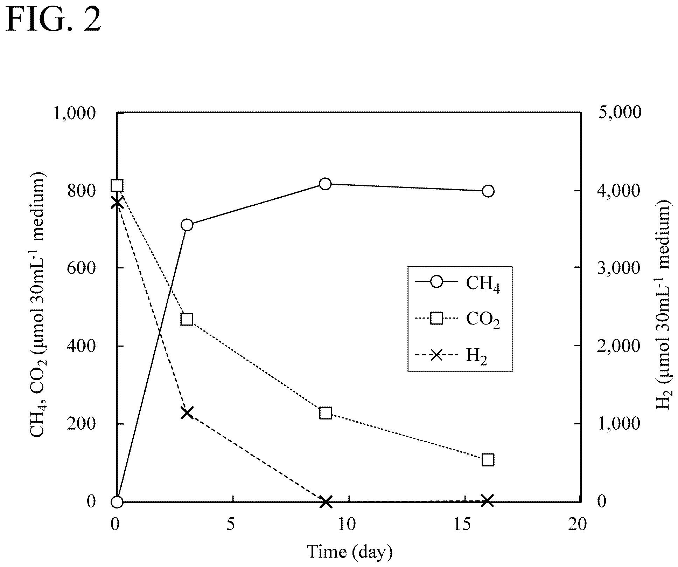

Fig. 2 is a graph showing an example of a gas analysis result according to the embodiment of the present invention.

In the culture tank 1, as shown schematically in Fig. 2, the concentrations of hydrogen gas, methane, and carbon dioxide change with time. Specifically, hydrogen gas and carbon dioxide are decreased by the hydrogen-utilizing methane generating bacteria, and methane is increased. From the analysis result of such a gas analysis device 4, it is possible to detect the generation state of methane.

Returning to Fig. 1, the liquid analysis device 5 is a microbial cell concentration measuring device that detects the existing amount of the microbial community in the culture liquid 20. Examples of a liquid analysis method by the liquid analysis device 5 include ion chromatography, liquid chromatography, mass analysis, and light absorbance measurement.

The liquid analysis device 5 of the present embodiment includes a light absorbance measuring device (a so-called turbidity measuring device for the culture liquid 20) that can obtain the analysis result relatively quickly in these liquid analysis methods.

The pH detector 6 detects a pH of the culture liquid 20 (that is, a hydrogen ion index).

The pH regulating agent input device 7 inputs a pH regulating agent (for example, sodium hydroxide or the like) into the culture liquid 20 on the basis of the detection result of the pH detector 6 to regulate the pH that is closer to acidity due to the generation of the methane to a pH that is optimum for growth of the methane generating bacteria.

The ORP detector 8 detects the ORP (that is, the oxidation-reduction potential) of the culture liquid 20.

The ORP regulating agent input device 9 inputs the reducing agent for maintaining an anaerobic state of the culture liquid 20 into the culture tank 1 on the basis of the detection result of the ORP detector 8.

The pH detector 6 and the ORP detector 8 constitute a culture liquid state detecting device 19 that detects the state of the culture liquid 20. The culture liquid state detecting device 19 may include a detector that detects the state amount of the culture liquid 20 in addition to the pH and the ORP.

The temperature sensor 10 detects a temperature of the culture tank 1.

The temperature control device 16 controls the temperature of the culture tank 1 on the basis of the detection result of the temperature sensor 10 to regulate the temperature to an optimum growth temperature for the methane generating bacteria. The optimum growth temperature differs depending on the bacterial species. Examples of the temperature control device 16 include a heater or the like that heats the culture tank 1 from the outside or the inside.

The pressure sensor 11 detects a pressure of the culture tank 1. As shown in the following Formula (3), the pressure of the culture tank 1 decreases with the generation of the methane, and thus the methane generation state can be detected from the pressure. In the generated gas extraction device 3 described above, the operation timing of the generated gas extraction device 3 may be controlled on the basis of the detection result of the pressure sensor 11.

Formula (3) CO₂ + 4H₂ → CH₄ + 2H₂O

The culture liquid input device 17 inputs a new culture liquid into the culture tank 1 while an anaerobic state is maintained.

If the gas phase portion of the culture tank 1 is in an anaerobic state, the culture liquid may be input to the gas phase portion.

Further, a new culture liquid may be input from the outside into a liquid phase portion of the culture tank 1 (that is, in the culture liquid 20).

The precipitate removing device 18 removes a precipitate generated in the culture liquid 20 from the culture tank 1. The precipitate includes metabolites of the methane generating bacteria and the like. The precipitate removing device 18 includes a mechanism that removes the precipitate from a drain pipe 1a of the culture tank 1. As a result, the precipitate can be removed from the bottom of the culture tank 1 using the existing equipment of the culture tank 1.

The data processor 13 stores and processes measurement data related to the culture environment of the culture tank 1 acquired from the various detectors described above (that is, the pH detector 6, the ORP detector 8, the temperature sensor 10, the pressure sensor 11, and the like).

The device controller 14 controls the operations of the various devices described above (that is, the gas analysis device 4, the liquid analysis device 5, the pH regulating agent input device 7, the ORP regulating agent input device 9, the temperature control device 16, the culture liquid input device 17, the precipitate removing device 18, and the like) on the basis of the processing result from the data processor 13.

Specifically, when the values acquired from the pH detector 6 and the ORP detector 8 are out of a range in which the methane generating bacteria can proliferate, the management device 15 adds a regulating agent for regulating the pH and the ORP to allow them to be held within a range in which the methane generating bacteria can proliferate.

Further, the management device 15 confirms a change with time (that is, a methane generation rate) in the methane generation amount from the measurement results of the gas analysis device 4 and the liquid analysis device 5 and evaluates a methane generation ability of microorganisms. The necessity for environmental regulation such as regulation of the microbial cell concentration and replacement of the culture liquid 20 is determined on the basis of the results.

According to the methane generating device having the above configuration, methane generation can be performed efficiently by considering not only the environmental conditions inside the culture tank 1 but also the active state of the methane generating bacteria. By converting carbon dioxide into methane in this way, it is possible to enable circulation of a carbon resource. In addition, by reusing the converted methane, it is possible to reduce the amounts of carbon resources such as petroleum, coal, and natural gas that are newly consumed. Furthermore, by recovering the heat generated during use, it is also possible to reuse heat energy.

Since the methane generating device generates methane by the biological method described above, the reaction temperature is lower than that of the chemical method of the related art, and low temperature exhaust heat of 200 °C or lower can be effectively utilized. In addition, by selecting an appropriate strain, it is possible to generate methane even at room temperature, and it is possible to suppress energy costs. Further, by using methane generating bacteria obtained locally (for example, from paddy soil, sediment of lakes and marshes, seafloor sediment, a deep aquifer of a thick sedimentary layer (that is, adduct) formed by subduction of oceanic plates, and the like), it is possible to suppress contamination of the environment due to microorganisms introduced from abroad.

In this way, in the present embodiment described above, a configuration in which the culture tank 1 in which the methane generating bacteria are cultured with the culture liquid 20, the gas feed device 2 that feeds carbon dioxide and hydrogen gas required by the methane generating bacteria to the culture tank 1, and the generated gas extraction device 3 that extracts methane generated by the methane generating bacteria are provided is employed. As a result, it is possible to obtain a methane generating device capable of generating methane that is a carbon resource that is useful as an energy source, and can be used as a raw material for chemical products by immobilizing carbon dioxide using hydrogen gas in a simple manner and while consuming little energy.

Although the preferred embodiment of the present invention has been described above with reference to the drawings, the present invention is not limited to the above embodiment. The shapes and combinations of the constituent members shown in the above-described embodiment are examples and can be variously changed on the basis of design requirements and the like without departing from the gist of the present invention.

For example, culture of microorganisms and generation of methane may be performed on the plate by immobilizing the methane generating bacteria in the culture tank 1 on a plate or the like.

Further, for example, carbon dioxide discharged from a thermal power plant, a factory, or the like may be used as a supply source of carbon dioxide.

Further, for example, hydrogen gas generated through electrolysis of water using renewable energy may be used as a supply source of hydrogen gas.

Further, for example, by-product hydrogen generated in a production process of a factory may be used as a supply source of hydrogen gas.

Further, for example, exhaust heat may be used for regulating the temperature of the culture tank 1.

Further, for example, when it takes a plurality of days (that is, N days (N is an integer of 2 or more)) to make all the introduced carbon dioxide into methane, methane may be obtained over time without any gap by operating N methane generating devices in parallel.

Further, for example, by causing a plurality of strains to coexist with the methane generating bacteria, a plurality of substances may be generated at the same time, or the environment may be maintained without active intervention.

### [Reference Signs List]

1 Culture tank
1a Drain pipe
2 Gas feed device
3 Generated gas extraction device
4 Gas analysis device
5 Liquid analysis device
6 pH detector
8 ORP detector (that is, oxidation-reduction potential detector)
9 ORP regulating agent input device (that is, reducing agent input device)
11 Pressure sensor
12 Stirring mechanism
13 Data processor
14 Device controller
16 Temperature control device
17 Culture liquid input device
18 Precipitate removing device
19 Culture liquid state detecting device
20 Culture liquid
21 Gas-liquid interface

## Claims

1. A methane generating device comprising:
a culture tank in which methane generating bacteria are cultured with a culture liquid;
a gas feed device that feeds carbon dioxide and hydrogen gas required by the methane generating bacteria to the culture tank; and
a generated gas extraction device that extracts methane generated by the methane generating bacteria.

2. The methane generating device according to claim 1, the methane generating device further comprising:
a temperature control device that controls a temperature of the culture tank.

3. The methane generating device according to claim 1 or 2, the methane generating device further comprising:
a gas analysis device that detects a generation state of the methane in the culture tank.

4. The methane generating device according to claim 3,
wherein, as the gas analysis device, a light absorption analysis device is provided.

5. The methane generating device according to any one of claims 1 to 4, the methane generating device further comprising:
a culture liquid state detecting device that detects a state of the culture liquid.

6. The methane generating device according to claim 5,
wherein, as the culture liquid state detecting device, a pH detector is provided.

7. The methane generating device according to claim 5,
wherein, as the culture liquid state detecting device, an oxidation-reduction potential detector is provided.

8. The methane generating device according to any one of claims 1 to 7, the methane generating device further comprising:
a liquid analysis device that detects an existing amount of a microbial community in the culture liquid.

9. The methane generating device according to claim 8,
wherein, as the liquid analysis device, a light absorbance measuring device is provided.

10. The methane generating device according to any one of claims 1 to 9, the methane generating device further comprising:
a pressure sensor that detects a pressure of the culture tank.

11. The methane generating device according to any one of claims 1 to 10, the methane generating device further comprising:
a precipitate removing device that removes a precipitate generated in the culture liquid from the culture tank.

12. The methane generating device according to claim 11,
wherein, as the precipitate removing device, a mechanism that removes the precipitate from a drain pipe of the culture tank is provided.

13. The methane generating device according to any one of claims 1 to 12, the methane generating device further comprising:
a culture liquid input device that inputs the culture liquid into the culture tank while an anaerobic state is maintained.

14. The methane generating device according to claim 13, the methane generating device further comprising:
a reducing agent input device that inputs a reducing agent into the culture tank in order to maintain an anaerobic state of the culture liquid.

15. The methane generating device according to any one of claims 1 to 14, the methane generating device further comprising:
a data processor that processes measurement data related to a culture environment of the culture tank; and
a device controller that controls the culture environment of the culture tank on the basis of a processing result from the data processor.

16. The methane generating device according to any one of claims 1 to 15, the methane generating device further comprising:
a mechanism that stirs and mixes the culture liquid and a mixed gas containing the carbon dioxide and the hydrogen gas.

17. The methane generating device according to any one of claims 1 to 16,
wherein the gas feed device feeds an inert gas to the culture tank before culture of the methane generating bacteria begins in the culture tank.

18. The methane generating device according to claim 15,
wherein the measurement data related to the culture environment of the culture tank is an oxidation-reduction potential of the culture liquid, and
wherein, when the oxidation-reduction potential is out of a range in which the methane generating bacteria can proliferate, the device controller adds a regulating agent for preparing the oxidation-reduction potential to the culture tank such that the oxidation-reduction potential is within a range in which the methane generating bacteria can proliferate.

19. The methane generating device according to claim 15,
wherein the measurement data related to the culture environment of the culture tank is a pH of the culture liquid, and
wherein, when the pH is out of a range in which the methane generating bacteria can proliferate, the device controller adds a regulating agent for preparing the pH to the culture tank such that the pH is within a range in which the methane generating bacteria can proliferate.

20. A methane generating method comprising:
feeding carbon dioxide and hydrogen gas required by methane generating bacteria to the culture tank in which the methane generating bacteria are cultured with a culture liquid; and
extracting methane generated by the methane generating bacteria.
